# EUROPEAN PATENT APPLICATION

(11) **EP 4 403 638 A2**
(43) Date of publication of application: **24.07.2024**
(21) Application number: 24172890.6
(22) Date of filing: 17.06.2016
(51) Int. Cl.: C12N 15/82

(54) **NOVEL CRISPR ENZYMES AND SYSTEMS**

(30) Priority: 18.06.2015 US 201562181739 P; 16.07.2015 US 201562193507 P; 05.08.2015 US 201562201542 P; 16.08.2015 US 201562205733 P; 24.09.2015 US 201562232067 P; 18.12.2015 US 201514975085; 07.01.2016 EP 16150428
(62) Divisional of application: 19171732.1
(71) Applicant: The Broad Institute Inc., Cambridge, MA 02142 (US); Massachusetts Institute of Technology, Cambridge, MA 02139 (US); President And Fellows Of Harvard College, Cambridge, Massachusetts 02138 (US)
(72) Inventor: ZHANG, Feng, Cambridge, MA 02139 (US); ZETSCHE, Bernd, Gloucester, MA 01930 (US); GOOTENBERG, Jonathan, S., Cambridge, MA 02138 (US); ABUDAYYEH, Omar, O., Cambridge, MA 02141 (US); SLAYMAKER, Ian, Cambridge, MA 02140 (US)
(74) Representative: De Clercq & Partners

(57) **Abstract**

The invention provides for systems, methods, and compositions for targeting nucleic acids. In particular, the invention provides non-naturally occurring or engineered DNA-targeting systems comprising a novel DNA-targeting CRISPR effector protein and at least one targeting nucleic acid component like a guide RNA. Methods for making and using and uses of such systems, methods, and compositions and products from such methods and uses are also disclosed and claimed.

## Description

The present invention relates to the creation of a packaging production dataset, in particular for a consumer product packaging, a method for producing a packaging and a computer system comprising a graphical user interface.

The possibilities regarding consumer product packaging have substantially increased throughout the last years. Many new production techniques, in particular printing techniques, have been proposed, and at the same time the expectations of consumers regarding the quality of packaging have increased. Furthermore, due to globalized markets, different requirements and different production quantities have to be realized in different countries according to local needs. To be competitive in the field of consumer products, it is further expected that frequent new designs, redesigns or amendments of the packaging design are carried out, for example in view of certain events or marketing campaigns. It is highly desirable to expedite the process from the decision for a new or amended design to having a packaging production dataset, which enables the production of the new packaging. Currently, this process may require several weeks until satisfactory completion.

However, the redesigning of packaging designs is work intensive and frequently leads to initial production delays, as many different requirements of the involved production machines have to be considered and implemented.

The present invention has the object of facilitating the creation of new consumer product packaging.

The present invention provides a computer-implemented method for creating a packaging production dataset, in particular for a consumer product packaging, wherein a database of pre-approved packaging features in different categories is provided, each approved packaging features having assigned metadata. When a selection of pre-approved packaging features is received, the selection of pre-approved packaging features is evaluated based on the metadata and a packaging production dataset is created, defining a packaging according to the pre-approved packaging features. By providing the database of pre-approved packaging features, the user is assisted in performing the technical task of defining and creating a packaging production dataset which ensures that the packaging production dataset is adequate to produce a corresponding packaging, or which at least ensures an efficient way of producing the desired packaging. The method can be completed instantly, if it requires the interaction of one user only, or at least in very few days, if it requires the interaction of several users or at least one measurement on a sample packaging, or both user interaction and at least one measurement. In contrast, in the prior art, the user would design a packaging production dataset based on his experience or memory. The prior art approach usually requires several iterations. Iterations lead to a loss of time, and an increase in cost and waste. In particular, the effects of the related production techniques or local requirements regarding the individual packaging features have to be evaluated. Additionally or alternatively, likely incompatibilities or imperfections have to be detected and resolved by adapting the packaging production dataset. In the prior art approach, this is particularly cumbersome in the field of consumer packaging desired to be frequently and swiftly changed in multiple markets worldwide. Frequently, such packaging needs design modifications for many different markets due to the different local consumer taste or legal requirements. In particular, this need for adaptation of the packaging to such boundary technical or regulatory conditions requires the creation of many different packaging production datasets that are closely related but slightly or significantly different from each other. For example, due to localized production, different production machines may be available in different production facilities. The different production machines may require different construction sequences. The different production machines may also require different parts of packaging material, for example packaging blanks that are printed or converted differently.

Due to different lot sizes, different production techniques may be recommendable. Due to different legal requirements, different features may have to be provided for the package. For example, it may be desired that a cardboard packaging comprises a certain 3D embossing structure as a design element, which is compatible with one printing technique, but which may lead to imperfections with another printing technique. The latter printing technique might be preferable for a version of the packaging for a smaller market.

A database of pre-approved packaging features is preferably an electronic library comprising different features which can be selected when designing a package. These features may be categorized in different categories to provide a better retrievability. The categories may relate to different aspects of the package. In particular, packaging features are categorized in at least one of the following exemplary categories: base material, packaging type, package forming, information provided on the package, design elements provided on the package, sealing and preservation features, additional marketing features.

Preferably, the database of pre-approved packaging features may be provided in a cloud-storage and the selection may be received in local terminals.

The metadata provided regarding each packaging feature is data, which is supplementary to the data specifying the feature itself. The metadata may relate, for example to one of the following parameters:
- to at least one production machine or converting operations with which the feature is producible,
- to restrictions regarding the feature such as for example size, color, form, content and/or manufacturing technique,
- to legal requirements regarding the feature, such as necessary information to be provided in context with a certain feature, etc.
- to one or more of the composition, physical and chemical properties, for example board thickness, coating, of the material,
- to a form of the feature, in particular 3D form or 2D shape,
- to a color or colors of the feature, in particular range or composition of the color or ink,
- to a material used for the feature,
- to alternatives regarding any of the above.

The packaging production dataset preferably comprises all data necessary for a respective production machine to produce a packaging defined by the selected packaging features.

With the inventive method, the metadata of the pre-approved packaging features allows an evaluation of the selection of the pre-approved packaging features and immediate feedback. Modification proposals or automatic modification (or both modification proposals and automatic modification) may be provided to avoid the creation of a packaging production dataset that is not immediately suitable for production of the desired packaging. Advantageously, this ensures that packaging features can only be combined for a packaging production dataset that do not lead to incompatibilities with the selected (and available) production machines.

Preferably, the method comprises the step of receiving a selection of at least one production machine for the packaging, in particular a converting machine, wherein the evaluation of the selection of pre-approved packaging features comprises evaluating their compatibility with the selected at least one production machine. The evaluation may be based on a comparison of a production machine dataset with a packaging feature, in particular the metadata thereof. In some embodiments, the selection is received directly from a user, in other embodiments it may be received as a result of further computer-implemented method steps, which determine a production machine depending on design or production requirements (or design and production requirements or other requirements) of the user. For example, if a heat-sealing machine is selected, then a compatibility evaluation regarding a heat sensitive packaging feature, at least in the regions of the packaging where heat sealing is actually applied, may be conducted. Other examples include the incompatibility of certain packaging features in the form of folds, creases or scores with certain printing production machines. This is advantageous, as the print may be destroyed by the application of these packaging features or may not be applicable to regions of the packaging where such features are present. The production of a packaging may involve at least one and preferably many converting operations. Throughout the document, the term "converting" is used to refer to an operation that changes one or more of appearance, shape, haptic and functionality of a packaging element. A packaging element may be for example a blank, a piece of cardboard, a foil, metallized paper or other materials suitable to be part of the packaging of an item. A possible converting operation is the application of color or surface structure or the application of color and surface structure, for example printing, coating or metalizing. Alternatively or additionally, a converting operation may be a form changing operation, in particular folding, creasing, scoring, embossing or hot foil stamping. Alternatively or additionally, a converting operation may be a separation operation, such as cutting, stamping, or scoring. Alternatively or additionally, a converting operation may be a joining operation, such as gluing or sealing.

In particular, several printing operations and according printing machines may be provided subsequently. For example highly customizable packaging features, such as text, may be printed with an adaptable printing method, such as digital printing, while prints which remain the same over more than one different packaging dataset, may be printed with less versatile, but more economic or precise methods, such as gravure printing.

The production machines involved in producing a folded cardboard packaging may be machines adapted to carry out one or more of the aforementioned converting operations. For example at least one of the production machines may be, a printing machine, a cutting and scoring machine, a folding machine, a gluing machine and a shrink-wrapping machine. Some, or all functionalities of the aforementioned functionalities may also be present in a single production machine. Further production machines, such as machines for the application of labels, application of inserts, onserts or outserts, or the application of surface treatments like for example embossing, flocking, metallizing or coating (or any combination thereof) may also be involved. In an exemplary production line at least some of the following production or converting operations are carried out: unwinding sheet material from a roll, flexography printing, digital printing, (rotary) embossing, slitting, rewinding, (digital) creasing, (digital cutting), (die) cutting and embossing.

Furthermore, the evaluation of the selection of the pre-approved packaging feature may comprise the evaluation of their compatibility with the production machines selected for the production of the respective packaging feature. For example, a user could be erroneously of the opinion that a certain print may be applicable with a certain printing machine or that a certain folding machine may be capable of providing a fold in a certain material. In these or other cases, the method may at least notify the user. The method may alternatively or in addition, automatically or semi-automatically (for example with the confirmation of the user) adapt the packaging dataset to fulfil the technical restrictions and the other requirements of the user. In particular, the production machines may be adapted for at least one of printing, cutting, folding or assembling of package components

In particular, the evaluation of pre-approved packaging features comprises checking the compatibility of at least two packaging features based on their metadata. For example, certain properties of a print may not be compatible with certain base materials (for example, one or more of film materials, plastics, paper or cardboard) which base materials have certain base materials properties (for example, surface roughness, electrical conductivity, hydrophobic properties, or coatings and similar). Furthermore, it may be checked whether a certain packaging type and size provides sufficient space for selected print designs. For example, a certain font size and type may be a prerequisite regarding design, but the amount of letters may be different in different translations. Thus, for certain geographical regions there may not be enough space for providing the text in the desired area of the package. According to the invention, this may be immediately revealed in the compatibility evaluation.

In one embodiment of the invention, the method comprises the step of storing the packaging production dataset and retrieving the packaging production dataset for the creation of a new packaging production dataset, which at least partially comprises packaging features of the retrieved packaging production dataset. This allows a user to quickly obtain or create new packaging production datasets, which are variations of previously existing packaging production datasets. This is particularly helpful if a similar design is for example used in several different markets, which have different languages or different legal requirements as to which information has to be provided on the package.

In particular, the method may comprise the step of receiving a new packaging feature created by a first user, submitting the packaging feature for approval to a second user and, if approved by the second user, storing the approved packaging feature as pre-approved packaging feature in the database. This enables to quickly build up a database of pre-approved packaging features. For example, the second user may be responsible for the technical implementation and, thus, will review whether the packaging feature could lead to any technical incompatibilities. The second user may modify the packaging feature or its metadata (or both the packaging feature and its metadata), such that technical incompatibilities may be avoided. In other embodiments, the first user may be a user concerned with the technical implementation and the second user may be a user who monitors other requirements, for example legal requirements or marketing requirements.

In particular, the packaging feature is one of a packaging type, a component of the package, at least one property of a print on the package, a brand, a text element, an arrangement of the at least one consumer good in the package, a type of consumer good arranged in the package and a designated market for the package. In the field of smoking articles, the packaging type may be for example a hinge-lid container, a soft pack container, a slide and shell container or a shoulder box container. In the field of smoking articles or aerosol generating articles, a component of the package may be an inner sealed package preserving the smoking articles. A property of a print on the package may be a glossy print or rough sections in the print. The packaging feature in the form of the brand of the product may lead to restrictions in certain other packaging features. For example, it may be mandatory that the brand is provided in a certain position or a specific size on the package. The packaging feature in the form of a text element may lead to the requirement of certain printing techniques, which are able to provide the text element in sufficient clarity.

In the field of packaging for rod-shaped consumer goods, such as smoking articles or aerosol generating articles, the packaging feature regarding the arrangement of the at least one consumer good in the package may, for example, be the collation. The collation describes in particular the numbers of rows and columns of rod-shaped consumer goods in a package and how these rows and columns are arranged in relation to each other, for example in a nested relationship. The collation determines how much space is left in the package in view of other packaging features. The type of consumer goods arranged in the package may define whether there are for example combustible smoking articles or heat-not-burn type aerosol generating articles arranged in the package. The type of consumer goods may impose certain limitations or requirements regarding for example the preservation of freshness or humidity of the respective articles or regarding security requirements. The packaging feature in the form of a designated market for the package may lead to restrictions in view of the packaging design. For example, in different jurisdictions, different information may have to be provided on the package, for example depending on the consumer goods comprised therein.

The evaluation of pre-approved packaging features in particular comprises evaluating their metadata regarding geographic approval. Thus, when creating a packaging feature, it may be manually approved for certain countries or jurisdictions and this information may be comprised in the metadata. Thus, if a user selects a packaging feature that is not approved for a certain jurisdiction, there may be a rejection of the selection or a modification of other packaging features. For example, the user may select the packaging feature of a holographic element to be provided on the package. However, in a certain jurisdiction, the feasible locations for providing such an element may be designated to comprise certain information on the product. Thus, while for some jurisdictions it may be possible to produce a package with the desired packaging feature, for other jurisdictions the respective machine producing the hologram may not be capable of producing the hologram in the available machine location. Thus, by evaluating the metadata regarding geographic approval, it is possible to avoid situations where production machines are instructed to produce features which these machines cannot produce.

In one embodiment, the method comprises producing a packaging based on the packaging production dataset, involving at least one converting operation, in particular at least one of printing, cutting, embossing, folding, creasing or scoring, receiving an approval of the packaging production dataset based on an evaluation of the produced packaging, and storing the packaging production dataset as an approved packaging production dataset in a database. Such method steps may in particular be beneficial, when it is not yet clear whether the packaging feature in the form of a certain print property or printing technique is compatible with the packaging feature of a certain packaging type or packaging material. Thus, by empirically evaluating the result of the desired packaging feature combination, it is possible to evaluate the compatibility of packaging features, which can be recorded in their respective metadata. Alternatively or additionally, the respective combination of packaging features can be stored as a packaging production dataset being indicated as approved in the database for further reference or as a template for future packaging production datasets with respective combinations of packaging features.

Alternatively or additionally, the step of producing the packaging based on the packaging production dataset may involve at least one of the steps of printing, cutting, embossing, folding, creasing or scoring. In a further step, an approval of the packaging production dataset based on the evaluation of the packaging is received and the packaging production dataset is stored as an approved packaging production dataset in the database. Thus, by empirical analysis of at least one packaging feature or, preferably, the effects of multiple packaging features with respect to each other, it is possible to determine pre-approved packaging production datasets. Alternatively or additionally, properties or information (or properties and information) obtained by empirical analysis may be recorded in the metadata of the respective packaging features.

In particular, the printed packaging is evaluated by measuring properties of the packaging using a measuring device, in particular a spectrophotometer, comparing the measured properties with a packaging feature and approving or rejecting the packaging feature. This enables for example ensuring the quality of color representation. The quality of color representation is highly important in today's consumer packaging, such that the package has a familiar appearance worldwide. In particular, different printing machines may have different printing qualities on different packaging materials. Preferably, a target value, for example a predefined color reference is provided, and further preferably a tolerance range regarding same. The measured property is preferably compared to the predefined color reference or the tolerance range. This enables to avoid subjectivity in the color evaluation. Carrying out the analysis as specified before, approved packaging features can be obtained which ensure a high quality of the print and its color representation.

Preferably, the method comprises the step of approving the packaging production dataset when all packaging features are either pre-approved packaging features or manually approved by a user. In a preferred embodiment, all packaging features have to be pre-approved packaging features. Where all packaging features are pre-approved, this advantageously lowers the user's workload and therefore increases the swiftness of creating the packaging production dataset. The pre-approval of all packaging features may also avoid potential human error.

The invention provides a method for producing a packaging, comprising a computer-implemented method for creating a packaging production dataset as specified above and further comprising the step of producing the packaging according to the packaging production dataset. In particular, a certain amount of identical packages are produced according to the packaging production dataset. Preferably, the step of producing the packaging comprises digital printing. Digital printing may include inkjet printing, laser printing, offset printing or other known printing techniques or combinations of these various printing techniques. Digital printing may apply color substance, in particular ink or toner, to a variety of substrates, such as at least one of: paper, cardboard, canvas, metalized paper, metalized cardboard, coated paper, coated cardboard, plastic and combinations thereof, for example a metallized plastic-cardboard laminate.

In some embodiments of digital printing, the color substance may not penetrate the substrate, but forms a thin layer on the substrate. Additional adhesion may be provided, for example by using a fuser fluid by a heating or curing process.

Digital printing allows high flexibility regarding new designs, as it can almost instantly print new designs in a predefined color gamut. In contrast, the more conventional printing technologies such as rotogravure, flexography and offset printing usually require the manufacture of equipment or tooling before a print can be carried out. However, most different types of printing and the according printing machines have restrictions that prevent the application of certain packaging features while enabling other packaging features.

The pre-approved packaging feature may also relate to design elements with variable data, such as design element which are unique to each package or only provide to a subset of packages. These design elements may be randomly generated, or comprised in lists. For example, each packaging may have a random generated artwork. Each package or subset of packages may comprise one slogan, name or brand from a list of a plurality of slogans, names or brands.

The invention further provides a computer system adapted to carry out a method as specified above, comprising a graphical user interface being adapted to displaying sets of pre-approved packaging features in different categories, wherein the computer system is adapted such that the selection of a user of a pre-approved packaging feature in one set reduces the number of selectable pre-approved packaging features in at least another set displayed on the graphical user interface, based on the compatibility of the pre-approved packaging features. For example, if a certain packaging type is selected, which enables only certain printing techniques, then packaging features that require other printing techniques are not displayed any more. If the packaging feature is a brand, then the selection of production-related or design-related packaging features may be limited. For example, for a certain brand, certain printing techniques, for example using soft touch lacquer may or may not be available.

When a certain production machine, for example a certain printing machine is selected, then certain other packaging features, such as high resolution graphic elements may or may not be available. There may be packaging features, which are production machine agnostic that is available for all productions machines, at least for certain production machine categories. For example, some design elements or artworks may be approved for all types of available printing machines. Some printing machines or techniques may not be compatible with other converting operations or machines. For example, embossing may not be available for certain types of print, as it may damage the print. All the aforementioned information may be comprised in the metadata of the packaging feature or the production machine dataset, or the packaging feature and the production machine dataset.

Thus, the user can be swiftly guided through a selection process towards the creation of a technically feasible packaging production dataset.

In particular, the graphical user interface may be adapted to enable the selection of available production machines, wherein the computer system is adapted such that the selection of a production machine reduces the number of selectable pre-approved packaging features on the graphical user interface. Thus, depending on the selected, respectively available, production machines, only packaging features that can be produced in high quality by the available production machine will be offered to the user for selection. The system may include the possibility of entering boundary conditions, such as at least one of the amount of packages, which shall be produced, the geographical location, the market for which the packages shall be produced, and the content of the packages. Thus, in dependency of these boundary conditions, only certain design elements and production machines are offered, which are approved for the respective market. The method may further limit the available options based on whether the chosen option is economically reasonable for the desired amount of packages to be produced. The method may also calculate or estimate a packaging unit cost based on the available or selected options so as to give the user the choice to determine the optimal or desired production run. The packaging unit cost may be displayed to the user.

Preferably, the computer system is adapted to display on the graphical user interface a preview of the packaging according to the pre-approved packaging features selected by a user. The preview may become more detailed with each selected pre-approved packaging features. Furthermore, the computer system may enable the user to de-select packaging features, for example if the system indicated that the selected packaging features are incompatible with other packaging features or with a selected or available production machine during the evaluation of the selection of the pre-approved packaging features.

In particular, the packaging production dataset is used for the production of containers for consumer goods, such as smoking articles or aerosol generating articles. Exemplary packaging features of such containers will be discussed in the following:
The container generally comprises an accessible inside and at least one container wall. For example, the container may be a pouch container, formed of a flexible sheet material, essentially being wrapped around a consumer good comprised therein, such as roll-your-own tobacco. Alternatively, the container may be a soft pack container, in the form of a rectangular cuboid, comprising flexible side walls and an openable portion at the top. Preferably, the container is a hinge lid container, comprising a cup shaped box and a lid, wherein the lid is hingedly connected to the box via a hinge line.

The terms "front", "back", "upper", "lower", "side", "top", "bottom" and other terms used to describe relative positions of the components of containers refer to the container in an upright position with the lid at the top end and the hinge on the back. The terms "left" and "right" are used with reference to side walls of the container when the container is viewed from the front in its upright position. When the container in the upright position is open, the goods contained in the box may be removed from the upper end of the container. The term "longitudinal" refers to a direction from bottom to top or vice versa. The term "transverse" refers to a direction perpendicular to the longitudinal direction across the front wall, the back wall or one of the side walls.

The term "hinge line" refers to a line about which the lid may be pivoted in order to open the container. A hinge line may be, for example, a fold line or a score line in the back wall of the container. Alternatively, a hinge line may be a fold line or a score line in a piece of material bridging the lower edge of the back wall of the lid and the upper edge of the back wall of the box. Such a piece of material may be, for example, a label that is permanently or removably attached to the back wall of the lid and the back wall of the box. Preferably, the hinge line is positioned along the back wall of the container at a level below the upper edge thereof.

Preferably, the container further comprises an inner frame mounted within the box, wherein the inner frame extends above the upper edges of at least the front wall of the box of the container. The inner frame is therefore visible to the consumer when the lid is opened. The front wall of the inner frame may be printed with indicia, which may be the same as, or different to the indicia printed on the front wall of the box. Alternatively, or in addition, the front wall of the inner frame may be cut into a distinctive shape, for example, to reflect the branding of the consumer goods. If required, the inner frame may also comprise a line of weakness to facilitate flattening of the container.

Preferably, the front wall of the inner frame is provided with a cut out portion at the upper edge thereof. This enables more convenient access to the consumer goods within the container, without significantly reducing the surface area of the front wall of the inner frame.

Alternatively, or in addition to an inner frame, the consumer goods within the container may be wrapped with an inner liner, which is visible above the upper edge of the front wall of the box and the front wall of the inner frame (if present) when the container is open.

The container may be in the shape of a rectangular parallelepiped, with right-angled longitudinal and right-angled transverse edges. Alternatively, the container may comprise one or more rounded longitudinal edges, rounded transverse edges, bevelled longitudinal edges or bevelled transverse edges, or combinations thereof. For example, the container may comprise, without limitation:
- One or two longitudinal rounded or bevelled edges on the front wall, and/or
- One or two longitudinal rounded or bevelled edges on the back wall.
- One or two transverse rounded or bevelled edges on the front wall, and/or
- One or two transverse rounded or bevelled edges on the back wall.
- One longitudinal rounded edge and one longitudinal bevelled edge on the front wall, and/or
- One transverse rounded edge and one transverse bevelled edge on the back wall.
- One or two transverse rounded or bevelled edges on the front wall and one or two longitudinal rounded or bevelled edges on the front wall.
- Two longitudinal rounded or bevelled edges on a first side wall or two transverse rounded or bevelled edges on the second side wall.

Where the container comprises one or more rounded edges and is made from a laminar blank, preferably the blank comprises three, four, five, six or seven scoring lines or creasing lines to form the rounded edge in the assembled container. The scoring lines or creasing lines may be either on the inside of the container or on the outside of the container. Preferably, the scoring lines or creasing lines are spaced apart from each other by between about 0.3 mm and 4 mm.

Preferably, the spacing of the creasing lines or scoring lines is in function of the thickness of the laminar blank. Preferably, the spacing between the creasing lines or scoring lines is between about 0.5 and about 4 times larger than the thickness of the laminar blank.

Where the container comprises one or more bevelled edges, preferably the bevelled one or more edges have a width of between about 1 mm and about 10 mm, preferably between about 2 and about 6 mm. Alternatively, the container may comprise a double bevel formed by three parallel creasing lines or scoring lines that are spaced apart such that two distinct bevels form on the edge of the container.

Alternatively to a container with a rectangular transverse cross section, the container may have a for example a polygonal cross section such as triangular, quadrangular or hexagonal, or a cross section which is oval, semi-oval, circular or semi-circular.

Where the container comprises a bevelled edge and is made from a laminar blank, the bevel may be formed by two parallel creasing lines or scoring lines in the laminar blank. The creasing lines or scoring lines may be arranged symmetrically to the edge between a first wall and a second wall. Alternatively, the creasing lines or scoring lines may be arranged asymmetrically to the edge between the first wall and the second wall, such that the bevel extends further into the first wall of the container than into the second wall of the container.

The container may be formed from any suitable materials including, but not limited to, cardboard, paperboard, plastic, metal, or combinations thereof. Preferably, the cardboard has a weight of between about 100 grams per square metre and about 350 grams per square metre.

The container may be used as a package for a variety of consumer goods. In particularly preferred embodiments, the container may be used to package smoking articles. The container may be advantageously used to package smoking articles including, but not limited to, known lit-end cigarettes, cigars or cigarillos. The container may be used to package heated aerosol generating articles comprising a combustible fuel element or heat source and an aerosol-generating substrate and aerosol generating articles for use with electrical systems such as IQOS^{™}.

Through an appropriate choice of the dimensions thereof, the container may be designed to hold different total numbers of consumer goods, or different arrangements of consumer goods. For example, through an appropriate choice of the dimensions thereof, the container may be designed to hold a total of between ten and thirty consumer goods.

The container may hold one, two, three, four or five separate bundles of consumer goods. The separate bundles may be arranged substantially parallel to the front wall and to the back wall or substantially perpendicular to the front wall and to the back wall.

Within a bundle, rod shaped articles may be arranged in different collations, depending on the total number of rod shaped articles, the dimensions of the rod shaped articles or the cross sectional shape of the container. For example, the rod shaped articles may be arranged in a bundle in a single row of five, six, seven, eight, nine or ten. Alternatively, the rod shaped articles may be arranged in two or more rows. The two or more rows may contain the same number of rod shaped articles. For example, the rod shaped articles may be arranged in: two rows of five, six, seven, eight, nine or ten; three rows of five, six, seven, eight, nine, or ten; or four rows of four, five, six or seven. Alternatively, the two or more rows may include at least two rows containing different numbers of smoking articles to each other. For example, the rod shaped articles may be arranged in: a row of five and a row of six (5-6); a row of six and a row of seven (6-7); a row of seven and a row of eight (7-8); a middle row of five and two outer rows of six (6-5-6); a middle row of five and two outer rows of seven (7-5-7); a middle row of six and two outer rows of five (5-6-5); a middle row of six and two outer rows of seven (7-6-7); a middle row of seven and two outer rows of six (6-7-6); a middle row of nine and two outer rows of eight (8-9-8); or a middle row of six with one outer row of five and one outer row of seven (5-6-7).

The container may hold filterless smoking articles and smoking articles with various filter tips. The container may hold rod shaped articles of differing length (for example, between about 40 mm and about 180 mm), diameter (for example, between about 4 mm and about 9 mm). In addition, the rod shaped articles may differ in strength of taste, resistance to draw and total particulate matter delivery. Wherein the container comprises more than one bundle, each bundle within the same container may hold the same or different types of rod shaped articles as listed above.

Preferably, the dimensions of the container are adapted to the length of the rod shaped articles, and the collation of the rod shaped articles. Typically, the outer dimensions of the container are between about 0.5 mm to about 5 mm larger than the dimensions of the bundle of rod shaped articles housed inside the container.

Preferably, the container may have a height of between about 60 mm and about 150 mm, more preferably a height of between about 70 mm and about 125 mm, wherein the height is measured from the top wall to the bottom wall of the container.

Preferably, the container may have a width of between about 12 mm and about 150 mm, more preferably a width of between about 70 mm and about 125 mm, wherein the width is measured from the first side wall to the second side wall of the container.

Preferably, the container may have a depth of between about 6 mm and about 100 mm, more preferably a depth of between about 12 mm and about 25 mm wherein the depth is measured from the front wall to the back wall of the container (comprising the hinge between box and lid).

Preferably, the ratio of the height of the container to the depth of the container is in between about 0.3 to 1 and about 10 to 1, more preferably between about 2 to 1 and about 8 to 1, most preferably between about 3 to 1 and 5 to 1

Preferably, the ratio of the width of the container to the depth of the container is in between about 1 to 1 and about 10 to 1, more preferably between about 2 to 1 and about 8 to 1, most preferably between about 2 to 1 and 3 to 1.

Preferably, the ratio of the height of the lid back wall to the height of the box back wall is between about 0 to 1 (hinge located at the top edge of the container) to about 1 to 1, more preferably, between about 1 to 5 and about 1 to 10, most preferably, between about 1 to 6 to about 1 to 8.

Preferably, the ratio of the height of the lid front wall to the height of the box front wall is between about 1 to 0 (lid covering the entire front wall) to about 1 to 10, more preferably, between about 1 to 1 and about 1 to 5, most preferably, between about 1 to 2 and about 1 to 3.

The exterior surfaces of the container may be printed, embossed, debossed or otherwise embellished with manufacturer or brand logos, trademarks, slogans and other consumer information and indicia. Alternatively, or in addition, the exterior surfaces of the container may be at least partially covered with lacquer, metalization, holograms, luminescent material, or any other materials that alter the feel, odour or appearance of the container.

Where the inner housing of the container contains one or more bundles of smoking articles, the smoking articles are preferably wrapped in an inner liner of, for example, metal foil or metalized paper.

Where the container comprises smoking articles or aerosol generating articles, the container may further comprise waste-compartments (for example for ash or butts) or other consumer goods, for example matches, lighters, extinguishing means, breath-fresheners or electronics. The other consumer goods may be attached to the outside of the container, contained within the container along with the smoking articles, in a separate compartment of the container or combinations thereof.

Once filled, the container may be shrink wrapped or otherwise over wrapped with a transparent polymeric film of, for example, high or low density polyethylene, polypropylene, oriented polypropylene, polyvinylidene chloride, cellulose film, or combinations thereof in a conventional manner. Where the container is over wrapped, the over wrapper may include a tear tape. The tear tape is preferably positioned around the container below the lower edge of the front wall of the lid, such that once the tear tape has been removed, the lid is free to be rotated about the first hinge line. Alternatively or in addition, there may be a second tear tape positioned around the container above the hatch, such that once the tear tape has been removed, the hatch is free to be rotated about the second hinge line. Alternatively, the tear tape may be provided lengthways around the container.

The present invention may enable the empirical evaluation of packaging features in the produced package and reflect the obtained information in the metadata. This may allow the quality of evaluation to increase. In particular, an increase in the quality of evaluation is achieved by evaluating the quality and consistency of the color in either sample packages produced separately for this purpose or sample products taken from an implemented production process. This can be done via an electronic platform that is connected with spectrometer devices or other hardware to enable the precise digitalization of colors. The electronic platform may be cloud based or otherwise accessible through a computer network. At least one of the following color properties may be obtained: color fingerprints, spectro-reflectance data, L*a*b*-values or transferable CxF-files. Unique color references may be stored with immediate access to the users. Thus, it is possible to provide objective color information all along the production line and implement quality assurance functions. These quality assurance functions enable to quickly detect deviations in color due to the combination of packaging features without having to rely on subjective assessments that may be prone to experience, light and other factors. Thus, the metadata can be adjusted and the printing result can be improved, meaning that digital quality control is enabled.

Consequently, a higher color consistency regarding different packaging features produced on different production machines can be obtained.

Exemplary embodiments of the invention will now be explained with reference to figures.
Fig. 1 shows a blank for a hinge-lid container and a hinge-lid container formed from same in accordance with pre-approved packaging features, according to an embodiment of the present invention.
Fig. 2 shows a symbolic representation of pre-approved packaging features, a packaging production dataset and production machines according to an embodiment of the present invention.
Fig. 3 shows steps of a method according to an embodiment of the present invention.
Fig. 4 shows a graphical user interface according to an embodiment of the invention during the selection of a packaging feature.
Fig. 5 shows a graphical user interface according to an embodiment of the invention showing information regarding a packaging production dataset.

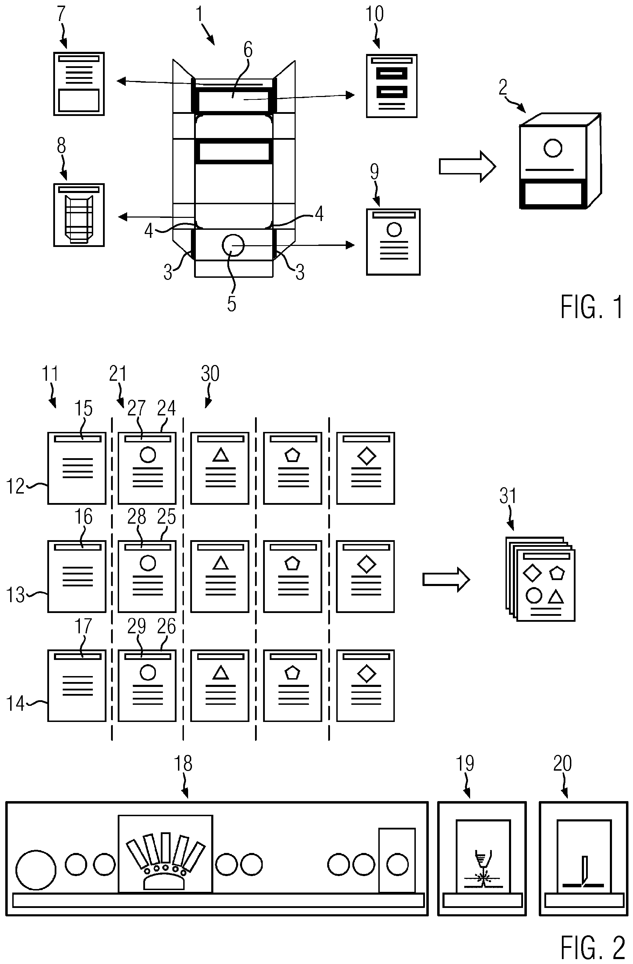

Fig. 1 shows a blank 1, preferably made of cardboard, and a container 2 which results from folding the blank along pre-determined scoring lines or creasing lines. As can be seen, the blank has a distinctive geometry. For example, the vertical edge line of the container is rounded by applying several parallel creasing lines 3 in one panel and rounded edges 4 in another panel. Furthermore, the blank 1 comprises brand representations 5 and text 6 due to legal requirements. Each of these elements or features is determined by a packaging feature, which is a set of data. The blank 2, in particular its material, geometry, and others, is determined by the packaging feature 7. The rounded edge feature, which comprises several parallel creasing lines 3 and according rounded edges 4, is determined by the packaging feature 8. The brand representation 5 is determined by the packaging feature 9. The text 6, in particular its font, size, content, and others, is determined by the packaging feature 10. As can be seen, many more packaging features are comprised in the blank, such as for example the distribution of glue or the thickness of the material. The method of the present invention enables producing the package without a very low likelihood of technical malfunctions or cumbersome trial and error periods in the production setup. For this purpose, a database of pre-approved packaging features is provided, which all have assigned metadata.

In Fig. 2, a schematic view of the logical data structure of the pre-approved packaging features is shown. For example, in a first category 11, the pre-approved packaging features 12, 13 and 14 are comprised, which define the packaging type. For example, hinge lid container as pre-approved packaging feature 12, soft pack container as pre-approved packaging feature 13 and pouch container as pre-approved packaging feature 14. Each of the pre-approved features comprises metadata 15, 16, 17, which define the compatibility of the pre-approved packaging feature with pre-approved packaging features from other categories and production machines 18, 19, 20. For example, the metadata 15 and 16 both indicate that a die cutting production machine 20 is compatible and required. For creating a package according to pre-approved packaging feature 14, a different production machine 18 may be necessary. Similarly, the metadata 15 and 16 may indicate that the production machine 19, for example a digital laser scoring machine, is compatible, while other scoring machines may not be compatible.

The next category 21 of pre-approved packaging features may relate to components of the package, such as an information leaflet 24, a sealed inner liner 25 or an electronic heating device 26. Now, the compatibility of these packaging features with packaging features 15, 16, 17 may be evaluated on the basis of the metadata 27, 28, 29 of the packaging features 23, 24, 25.

In a further category 30 of packaging features, marketing information may be defined. The user now selects a combination of pre-approved packaging features, for example images or copy lines from a marketing campaign, and, according to the embodiment of the inventive method, the selection of the pre-approved packaging features is evaluated based on the metadata. If there are no objections in the combinations, a packaging production dataset 31 is created. Otherwise, an automatic modification of packaging features may be effected or a selection may simply be refused. Also, it is possible to only provide those pre-approved packaging features to the user for selection that are compatible with the previously selected features.

Other production machines may, for example, be folding and blank cutting machines that are required to manufacture a container as shown in Fig. 1.

The production machine may then be provided with the packaging production dataset and produce a packaging with the specified features. Depending on the embodiment of the invention, this may still be a packaging for final approval or immediately a packaging for retail. If it is a packaging for approval, further analytics may be carried out, such as measuring properties of the print on the packaging to pre-approve further combinations of packaging features, that is, adapt the metadata accordingly or confirm assumptions reflected in the metadata.

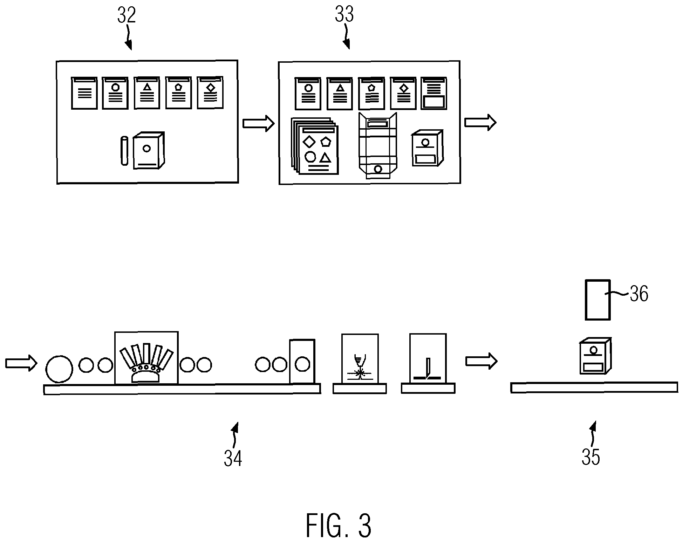

Fig. 3 shows an embodiment of the method according to the invention. In a first step 32, packaging features are provided for selection in a digital catalogue. Optionally, additional packaging features may be added to or may be removed from the catalogue by the user. The catalogue may comprise pre-approved packaging features and approved packaging production datasets. By selecting pre-approved packaging features or approved packaging production datasets, or pre-approved packaging features and approved packaging production datasets from the catalogue, a new packaging production dataset may be produced in a second step 33. The selection is evaluated based on the metadata of the selected packaging features, and a packaging production dataset is created. The packaging production dataset may be approved with or immediately after its creation. In a third step 34, one or several packages are produced according to the packaging production dataset. In a fourth step 35, the package or features of the packaging are analyzed by means of a measuring device 36. The result may be used to approve packaging features or the whole packaging production dataset. While this embodiment of the method is rather linear, there may be a reiteration of steps. For example, after the measurement in step 26 indicated an incompatibility of packaging features or of a packaging feature and a production machine, the packaging feature or the selection of the production machine may be amended in step 32.

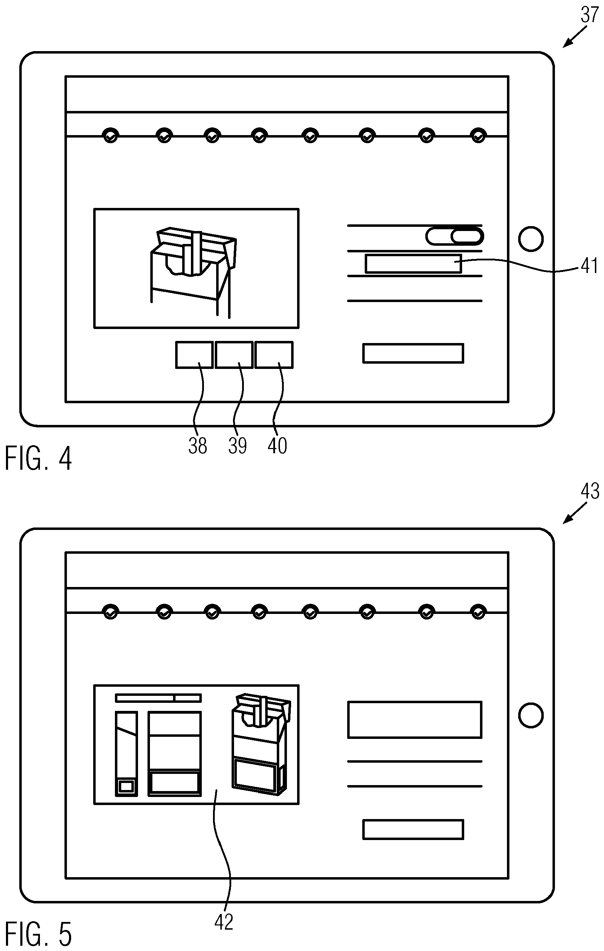

Fig. 4 shows a graphical user interface 37 for a computer system according to an embodiment of the present invention. The information currently on display allows the user to select a pre-approved packaging feature in the form of a component of the package, namely an inner liner. As can be seen, several different options 38, 39, 40 are available for the inner liner. The user may be provided with supplementary information 41 on the cost and properties of the selected packaging feature. Once a user has selected all properties, the packaging production dataset is displayed as a preview 42 in the graphical user interface 43 of Fig. 4. The preview 42 visualizes selected packaging features and a selection option for the user to create the packaging production dataset. The packaging production dataset is then saved and may be provided to one or several production machines that produce a corresponding package.

The present invention is particularly beneficial for consumer goods, which require frequent design modifications, due to marketing reasons or legal requirements. Furthermore, it is beneficial for consumer goods, which have to be provided with different packages for different markets, due to marketing reasons or legal requirements. This requires that new types of packaging are quickly implemented in a short amount of time and often for very different production machines depending on the amount of packages to be produced. For example, smaller lot sizes may be beneficially produced through digital printing. Digital printing typically has a higher printing cost per package than conventional printing methods for large production runs, but may be more cost efficient, for smaller production runs. Digital printing is generally more flexible regarding design changes. Depending on the different requirements in the selected package, the printing method may not be technically compatible with other packaging features. Thus, if for example a package is modified by changing the packaging feature of the printing technique and the amount of information provided on the package for a specific market, the inventive method may indicate that this modified selection is not feasible and may propose or automatically carry out amendments in other packaging features. The present invention therefore relies on an online catalogue of pre-approved packaging features and pre-approved packaging production datasets, corresponding to pre-approved products, which may correspond to packages previously produced by the inventive method. Thus, modifications on already available products and the creation of new packaging products may be swiftly carried out.

The database comprises packaging features with high resolution, detailed information regarding the packaging features. This enables providing the detailed information required for instructing the respective production machine. The database may also comprise lower quality preview information, which may be provided to the user during the selection process. The metadata may not only relate to the packaging but may also define encoding standards, provenance, design ownership, access rights or location of original creation. It may enable tracing back the packaging features to their creator in case of a need for further evaluation or implementation. It will provide information on the production machines, tools or systems used for creation of the packaging and how it should be handled and displayed. The metadata may comprise information on the communication interface of production machines required for the production of the packaging feature. Thus, it may enable the operation of the production machine in a standardized manner from a common user interface for many production machines.

Thus, all packaging assets and labeling information may be comprised within the pre-approved packaging features. Consequently, it enables simplifying and standardizing packaging development by assembling selected pre-approved packaging features together according to pre-defined parameters and design templates. Thus, packaging production datasets can be created in very short time.

## Claims

1. A computer-implemented method for creating a packaging production dataset, in particular for a consumer product packaging, comprising the steps of:
providing a database of pre-approved packaging features in different categories, each having assigned metadata,
receiving a selection of pre-approved packaging features,
evaluating the selection of pre-approved packaging features based on their metadata, and
creating a packaging production dataset defining a packaging according to the pre-approved packaging features.

2. The method according to claim 1, comprising the step of receiving a selection of at least one production machine for the packaging, wherein the evaluating of the selection of pre-approved packaging features comprises evaluating their compatibility with the selected at least one production machine.

3. The method according to claim 1 or 2, wherein the evaluating of the pre-approved packaging features comprises checking the compatibility of at least two packaging features based on their metadata.

4. The method according to any one of the previous claims, comprising the steps of storing the packaging production dataset, and retrieving the packaging production dataset for creation of a new packaging production dataset, which at least partially comprises packaging features of the retrieved packaging production dataset.

5. The method according to any one of the previous claims, comprising the step of receiving a new packaging feature created by a first user, submitting the packaging feature for approval to a second user, and, if approved by the second user, storing the approved packaging feature as a pre-approved packaging feature in the database.

6. The method according to any one of the previous claims, wherein the packaging feature is one of a packaging type, a component of the package, at least one property of a print on the package, a brand, a text element, an arrangement of the at least one consumer good in the package, a type of consumer good arranged in the package and a designated market for the package.

7. The method according to any one of the previous claims, wherein the evaluating of the pre-approved packaging features comprises evaluating their metadata regarding geographic approval.

8. The method according to any one of the previous claims, further comprising the steps of:
producing a packaging based on the packaging production dataset, involving at least one of the converting operations of printing, cutting, embossing, folding, creasing or scoring,
receiving an approval of the packaging production dataset based on an evaluation of the produced packaging, and
storing the packaging production dataset as an approved packaging production dataset in a database.

9. The method according to any one of the previous claims, comprising evaluating the printed packaging by
measuring properties of the print on the packaging using a measuring device, in particular a spectrophotometer,
comparing the measured properties with a packaging feature defining color, and approving the packaging feature.

10. The method according to any one of the previous claims, further comprising the step of approving the packaging production dataset, when all packaging features are either pre-approved packaging features or manually approved by a user.

11. A method for producing a packaging, comprising a computer-implemented method for creating a packaging production dataset according to any one of the preceding claims, and further comprising the step of producing the packaging according to the packaging production dataset.

12. The method according to any one of the previous claims, wherein the step of producing the packaging comprises digital printing.

13. A computer system adapted to carry out a method according to any one of the preceding claims and/or comprising a graphical user interface,
the graphical user interface being adapted to display sets of pre-approved packaging features in different categories,
wherein the computer system is adapted so that the selection by a user of a pre-approved packaging feature in one set reduces the number of selectable pre-approved packaging features in at least another set displayed on the graphical user interface, based on the compatibility of the pre-approved packaging features.

14. The computer system of claim 13, wherein the graphical user interface is adapted to enable the selection of available production machines, wherein the computer system is adapted so that the selection by a user of a production machine reduces number of selectable pre-approved packaging features on the graphical user interface.

15. The computer system of claim 13 or 14, being adapted to display on the graphical user interface a preview of the packaging according to the pre-approved packaging features, selected by a user.
